# EUROPEAN PATENT APPLICATION

(11) **EP 4 772 117 A1**
(43) Date of publication of application: **08.07.2026**
(21) Application number: 25222926.5
(22) Date of filing: 12.12.2025
(51) Int. Cl.: A61B 17/04

(54) **LOCKING DEVICE AND TISSUE LIGATION APPARATUS HAVING SUCH LOCKING DEVICE**

(30) Priority: 03.01.2025 CN 202510011929
(71) Applicant: Micro-Tech (Nanjing) Co., Ltd., Nanjing, Jiangsu 210032 (CN)
(72) Inventor: WANG, Yaoyuan, Nanjing (CN); Binmoeller, Kenneth F., Nanjing (CN); XIE, Lei, Nanjing (CN); JIN, Hongyan, Nanjing (CN); HU, Jie, Nanjing (CN); XI, Jiefeng, Nanjing (CN)
(74) Representative: Monteiro Alves, Inês

(57) **Abstract**

Disclosed are a locking device, a tissue ligation apparatus, and an endoscopic surgical instrument, belonging to the technical field of medical devices. In the embodiment of the present invention, a locking member and an elastic member are provided in an inner cavity of a housing of the locking device, the elastic member is capable of generating an elastic force due to its own elastic deformation, and the elastic force is applied to the locking member to continuously press the locking member. The first threading hole and the second threading hole of the housing, and the threading channel of the locking member, can be configured to thread the medical wire, so that the locking member, being pressed by the elastic force of the elastic member, can press the medical wire, which is threaded through the threading channel, against the housing, thereby fixing the medical wire and realizing the locking of the medical wire. Moreover, since the elastic force of the elastic member continuously acts on the locking member, it can continuously press the medical wire, reducing the occurrence of loosening caused by tissue peristalsis or tissue elasticity, and improving surgical efficiency and the success rate of surgery.

## Description

### TECHNICAL FIELD

The present invention belongs to the technical field of medical devices, and specifically relates to a locking device, a tissue ligation apparatus, and an endoscopic surgical instrument.

### BACKGROUND ART

In some surgeries, it is necessary to use a locking device to lock wire loops, mesh baskets, or the like. For example, in digestive tract surgery, when it is required to ligate a tumor or tissue, the wire loop must be locked. However, the current locking methods have certain problems. During the tightening process, due to the peristalsis or elasticity of tissue, the wire loop may rebound and become loose. Loosening of the wire loop can cause instrument failure, reduce surgical efficiency, and increase the duration of surgery. In addition, it can also lead to perforation, rupture, and increase the difficulty of surgery, thereby aggravating patient pain, and raising the risk of patient death.

### SUMMARY

The objectives of the present invention are as follows. An embodiment of the present invention provides a medical locking device to solve the technical problem that loosening may occur when locking a medical wire; another objective of the embodiment of the present invention is to provide a tissue ligation apparatus; and another objective of the embodiment of the present invention is to provide an endoscopic surgical instrument.

The technical solution is as follows. An embodiment of the present invention provides a locking device for locking a medical wire. The locking device comprises:
a housing provided with an inner cavity, a first threading hole, and a second threading hole, wherein the first threading hole and the second threading hole are respectively in communication with the inner cavity;
a locking member arranged in the inner cavity, wherein the locking member is provided with a threading channel, and the first threading hole, the threading channel, and the second threading hole are configured for the medical wire to pass through; and
an elastic member arranged in the inner cavity and connected with the housing and the locking member, wherein the elastic member is arranged to apply an elastic force to the locking member, so that the locking member is able to press the medical wire against the housing.

In some embodiments, the locking member is provided with a plurality of threading channels, and the locking member is provided to be subjected to the elastic force so as to press the medical wire threaded in each threading channel against the housing.

In some embodiments, the elastic member is arranged to apply an elastic force along a first direction to the locking member, so that the locking member is able to press the medical wire against a first inner wall of the housing; and
the threading channel is provided with a channel opening, the channel opening is formed on one side of the locking member away from the elastic member, and in the first direction, at least a portion of the channel opening is formed toward the first inner wall.

In some embodiments, the locking member comprises a first outer wall away from the elastic member and facing the first inner wall, a channel opening is arranged on the first outer wall, and both the first outer wall and the first inner wall are inclined relative to the first direction.

In some embodiments, the second threading hole is located on one side of the locking member away from the elastic member; and
the first inner wall is arranged on one side of a hole wall of the second threading hole close to the locking member and is connected with the hole wall, and the first inner wall extends obliquely in a direction away from the second threading hole and close to the elastic member.

In some embodiments, the locking member is provided to be subjected to the elastic force, and one end of the locking member away from the elastic member is capable of at least partially extending into the second threading hole.

In some embodiments, the housing is provided with a plurality of second threading holes, and the number of the second threading holes corresponds to the number of the threading channels.

In some embodiments, the inner cavity has a tapered section formed by the first inner wall, and along the first direction, a size L₁ of the tapered section gradually decreases; and
the locking member comprises a tapered portion, at least a portion of the tapered portion is located within the tapered section, and along the first direction, a size L₂ of the tapered portion gradually decreases, with the first outer wall arranged on the tapered portion.

In some embodiments, an angle between the first outer wall and the first direction is α, and an angle between the portion of the first inner wall opposite to the first outer wall and the first direction is β, satisfying β≥α.

In some embodiments, the threading channel extends linearly along the first direction.

In some embodiments, the first threading hole, the threading channel, and the second threading hole are sequentially arranged along the first direction, the second threading hole has an axis extending along the first direction, and the plurality of threading channels are arranged around a periphery of the axis.

In some embodiments, the first threading hole, the inner cavity, and the second threading hole are coaxially arranged along the first direction.

In some embodiments, the housing is provided with a first guiding portion, the locking member is provided with a second guiding portion matched with the first guiding portion, and the second guiding portion and the first guiding portion are in guiding cooperation to guide the locking member along the first direction.

In some embodiments, among the first guiding portion and the second guiding portion, one is provided as a guiding groove, the other is provided as a guiding protrusion, and the guiding protrusion is movably provided within the guiding groove.

In some embodiments, the housing comprises a cover portion and a main body portion, the cover portion and the main body portion are connected to form the inner cavity, the first threading hole is provided in the cover portion, and the second threading hole is provided in the main body portion.

In some embodiments, the elastic member has a first end and a second end opposite to each other along the first direction, the first end abuts against the cover portion, and the second end abuts against the locking member.

In some embodiments, the locking member is provided with a flange portion, and the second end abuts against the side wall of the flange portion facing the cover portion.

In some embodiments, the housing has a shape selected from a cylinder or a prism.

Accordingly, the tissue ligation apparatus provided by the embodiment of the present invention comprises:
the locking device according to any of the embodiments described above; and
a medical wire, wherein the medical wire is threaded through the first threading hole, the threading channels, and the second threading hole of the locking device.

In some embodiments, the medical wire comprises:
a first pulling strand threaded through the first threading hole, one of the threading channels, and the second threading hole;
a second pulling strand threaded through the first threading hole, another of the threading channels, and the second threading hole; and
a wire loop arranged on one side of the second threading hole away from the threading channels and connecting to the first pulling strand and the second pulling strand.

Accordingly, the endoscopic surgical instrument, provided by the embodiment of the present invention, includes the locking device according to any of the embodiments described above, or includes the tissue ligation apparatus according to any of the embodiments described above.

The beneficial effect is as follows. An embodiment of the present invention provides a locking device for locking a medical wire. The locking device includes: a housing provided with an inner cavity, a first threading hole, and a second threading hole, wherein the first threading hole and the second threading hole are respectively in communication with the inner cavity; a locking member arranged in the inner cavity, wherein the locking member is provided with a threading channel, and the first threading hole, the threading channels, and the second threading hole are configured for the medical wire to pass through; and an elastic member arranged in the inner cavity and connected with the housing and the locking member respectively, wherein the elastic member is arranged to apply an elastic force to the locking member, so that the locking member is able to press the medical wire against the housing. In the embodiment of the present invention, a locking member and an elastic member are provided in an inner cavity of a housing of the locking device, the elastic member is capable of generating an elastic force due to its own elastic deformation, and the elastic force is applied to the locking member to continuously press the locking member. The first threading hole and the second threading hole of the housing, and the threading channels of the locking member, can be configured for threading the medical wire, so that the locking member, being pressed by the elastic force of the elastic member, can press the medical wire, which is threaded through the threading channel, against the housing, thereby fixing the medical wire and realizing the locking of the medical wire. Moreover, since the elastic force of the elastic member continuously acts on the locking member, it can continuously press the medical wire, reducing the occurrence of loosening caused by tissue peristalsis or tissue elasticity, and improving surgical efficiency and the success rate of surgery.

An embodiment of the present invention provides a tissue ligation apparatus, comprising the locking device according to any one of the above embodiments and a medical wire, wherein the medical wire is threaded through the first threading hole, the threading channels, and the second threading hole of the locking device. The tissue ligation apparatus can include all the technical features and technical effects of the locking device described above, and therefore, details will not be repeated herein.

The endoscopic surgical instrument of the embodiment of the present invention can include all the technical features and technical effects of the locking device or the tissue ligation apparatus described above, and details will not be repeated herein.

### BRIEF DESCRIPTION OF DRAWINGS

In order to more clearly illustrate the technical solutions of the embodiments of the present invention, the drawings used in the embodiments are briefly introduced below. It is apparent that the drawings described below are merely some embodiments of the present invention, and those skilled in the art can obtain other drawings based on these drawings without inventive efforts.
FIG. 1 is a schematic structural view of a locking device provided in some embodiments of the present invention;
FIG. 2 is a schematic structural view of a locking device provided in some embodiments of the present invention from another angle;
FIG. 3 is a front view schematic structural view of a locking device provided in some embodiments of the present invention;
FIG. 4 is a sectional schematic structural view along line A-A in FIG. 3;
FIG. 5 is a three-dimensional schematic structural view along line A-A in FIG. 3;
FIG. 6 is a sectional schematic structural view along a plane perpendicular to the cross section in FIG. 4 and passing through axis OO';
FIG. 7 is a three-dimensional schematic structural view of FIG. 6;
FIG. 8 is a schematic structural view of a locking member in some embodiments of the present invention;
FIG. 9 is a sectional schematic structural view of a locking member in some embodiments of the present invention;
FIG. 10 is a schematic structural view of a locking device with medical wires threaded in some embodiments of the present invention;
FIG. 11 is a sectional schematic structural view of a locking device with the medical wires threaded in other embodiments of the present invention;
FIG. 12 is a sectional schematic structural view of a locking device with the medical wires threaded in other embodiments of the present invention;
FIG. 13 is a schematic structural view of a locking device provided in some other embodiments of the present invention;
FIG. 14 is a side view schematic structural view of a locking device provided in some other embodiments of the present invention;
FIG. 15 is a sectional schematic structural view along line C-C in FIG. 14;
FIG. 16 is a front view schematic structural view of a locking device provided in some other embodiments of the present invention;
FIG. 17 is a sectional schematic structural view along line B-B in FIG. 16;
FIG. 18 is a schematic structural view of a cover portion in some other embodiments of the present invention;
FIG. 19 is a schematic structural view of a main body portion in some other embodiments of the present invention;
FIG. 20 is a perspective schematic structural view of a locking device provided in some embodiments of the present invention;
FIG. 21 is a sectional schematic structural view along line D-D in FIG. 20;
FIG. 22 is a sectional schematic structural view along line E-E when a locking device in FIG. 20 is threaded with the medical wires;
FIG. 23 is a perspective schematic structural view of a locking device provided in some embodiments of the present invention;
FIG. 24 is a sectional schematic structural view along line F-F in FIG. 23;
FIG. 25 is a perspective schematic structural view of a locking device provided in some embodiments of the present invention; and
FIG. 26 is a sectional schematic structural view along line G-G in FIG. 25.

Reference numerals: 10-locking device; 100-housing; 110-inner cavity; 111-tapered section; 120-first threading hole; 130-second threading hole; 140-cover portion; 150-main body portion; 160-first inner wall; 170-first guiding portion; 200-locking member; 210-threading channel; 211-channel opening; 220-first outer wall; 230-tapered portion; 240-flange portion; 250-second guiding portion; 300-elastic member; 310-first end; 320-second end; 20-medical wire; 21-first pulling strand; 22-second pulling strand; 23-wire loop; X-first direction; OO'-axis.

### DETAILED DESCRIPTION OF EMBODIMENTS

The following description will combine the drawings in the embodiments of the present invention to provide a clear and comprehensive explanation of the technical solution in the embodiments of the present invention. Obviously, the described embodiments are only a part of the embodiments of the present invention, and not all of them. Based on the embodiments in the present invention, all other embodiments obtained by those skilled in the art without making inventive efforts are within the scope of protection of the present invention.

In the description of the present invention, it should be understood that the terms "proximal" and "distal" are referenced to the surgical operator (physician), where "proximal" refers to the end of the surgical instrument closer to the operator, and "distal" refers to the end farther from the operator, that is, the end closer to the patient. The terms "upper", "lower", "inner", "outer", and the like indicating orientation or positional relationships are based on the orientation or positional relationships shown in the drawings. These terms are merely intended to facilitate the description of the present invention and simplify the description and are not intended to indicate or imply that the referenced devices or components must have a specific orientation, be constructed in a specific orientation, or operate in a specific orientation. Therefore, these terms should not be construed as limiting the present invention. The term "a plurality of" means two or more, and "at least one" refers to one, two, or more, unless otherwise explicitly and specifically limited. The terms "mount", "connect", and "link" should be understood in a broad sense. For example, they may refer to a fixed connection or a detachable connection, or an integral connection; they may be directly connected or indirectly connected through an intermediate medium; they may refer to communication within two devices or an interaction relationship between two devices, unless otherwise explicitly and specifically limited. The terms "first" and "second" are used for descriptive objectives only and should not be understood as indicating or implying relative importance or specifying the quantity of the indicated technical features. Therefore, features limited by "first" and "second" may explicitly or implicitly include one or more of such features.

In the description of the present invention, the first direction is introduced to facilitate the explanation of the relative positional relationships among the components of the locking device, wherein the first direction is the direction in which the elastic member applies elastic force to the locking member in the locking device. In the drawings of the present invention, the first direction X is indicated by an arrow labeled X.

As a medical instrument, the locking device can be applied in various types of surgeries to lock medical wires, so as to ensure smooth surgical operation and improve the success rate of surgery. For example, the locking device can be applied to lock a wire loop in tissue ligation surgery, can be applied to lock a mesh basket in stone removal surgery, and can also be applied to lock a suture in suturing surgery. In the related technology, medical wires are directly bound by structures such as silicone tubes, and in this manner, when affected by tissue peristalsis or tissue elasticity, the silicone tube will move backward, causing the originally fastened wire loop to loosen.

In view of the foregoing, the present invention provides an embodiment of a locking device 10. The locking device 10 can firmly lock a medical wire 20 and can be used in various types of surgeries such as tissue ligation, vessel closure, stone removal, and suturing.

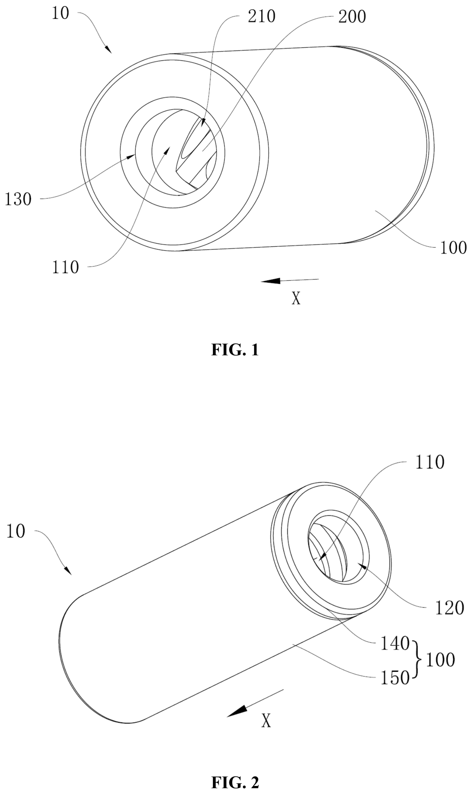

Refer to FIGS. 1 to 10, the locking device 10 according to the embodiment of the present invention includes a housing 100, a locking member 200, and an elastic member 300. The housing 100 serves as an outer shell structure of the locking device 10. The locking member 200 and the elastic member 300 are accommodated inside the housing 100, and the three cooperate to achieve locking of the medical wire 20.

The housing 100 provides an inner cavity 110, a first threading hole 120, and a second threading hole 130. The first threading hole 120 and the second threading hole 130 are respectively in communication with the inner cavity 110. The inner cavity 110 is a space formed inside the housing 100. The space is configured to accommodate the locking member 200 and the elastic member 300, and allows the medical wire 20 to pass through. The first threading hole 120 and the second threading hole 130 are holes formed in the housing 100 and communicated with the inner cavity 110. The two are respectively in communication with the inner cavity 110, thereby forming a continuous passage. The medical wire 20 can enter the inner cavity 110 from the first threading hole 120 and exit from the second threading hole 130, or can enter the inner cavity 110 from the second threading hole 130 and exit from the first threading hole 120.

The locking member 200 is arranged in the inner cavity 110. The locking member 200 has a threading channel 210. After the medical wire 20 enters the inner cavity 110, it can pass through the threading channel 210. The elastic member 300 is arranged in the inner cavity 110 and is respectively connected with the housing 100 and the locking member 200. The elastic member 300 is arranged to apply an elastic force to the locking member 200, so that the locking member 200 can press the medical wire 20 against the housing 100. The locking member 200 is movably arranged in the inner cavity 110 of the housing 100. The elastic member 300 connects to and presses the locking member 200, so that the locking member 200 can move along the direction of the elastic force, or has a tendency to move along the direction of the elastic force, thereby pressing the medical wire 20, which is threaded in the threading channel 210, against the housing 100 to fix the medical wire 20.

In the locking device 10 of the embodiment of the present invention, by arranging the locking member 200 and the elastic member 300 in the inner cavity 110 of the housing 100, the elastic member 300 can generate an elastic force due to its own elastic deformation and apply the elastic force to the locking member 200. That is to say, the elastic member 300 can be disposed in a compressed state, so that it can apply an elastic force to the locking member 200 to continuously press the locking member 200. The first threading hole 120 and the second threading hole 130 of the housing 100, and the threading channels 210 of the locking member 200, can be configured for threading the medical wire 20, so that the locking member 200, being pressed by the elastic force of the elastic member 300, can press the medical wire 20, which is threaded through the threading channel 210, against the housing 100, thereby fixing the medical wire 20 and realizing the locking of the medical wire 20. Moreover, since the elastic force of the elastic member 300 continuously acts on the locking member 200, it can continuously press the medical wire 20, reducing the occurrence of loosening caused by tissue peristalsis or tissue elasticity, and improving surgical efficiency and the success rate of surgery.

In addition, by providing the threading channel 210 in the locking member 200, the space occupied by the medical wire 20 in the inner cavity 110 is reduced, and the space of the inner cavity 110 can be appropriately reduced. In this way, on one hand, the structural volume of the locking device 10 can be reduced, so that the locking device 10 can more conveniently pass through the forceps channel of other medical instruments (such as an endoscopic), improving operational convenience; and on the other hand, the space redundancy in the inner cavity 110 is eliminated, the shaking of the locking member 200 in the inner cavity 110 is avoid, and the locking member 200 can be more stably assembled in the inner cavity 110, thereby improving the stability of the locking device 10.

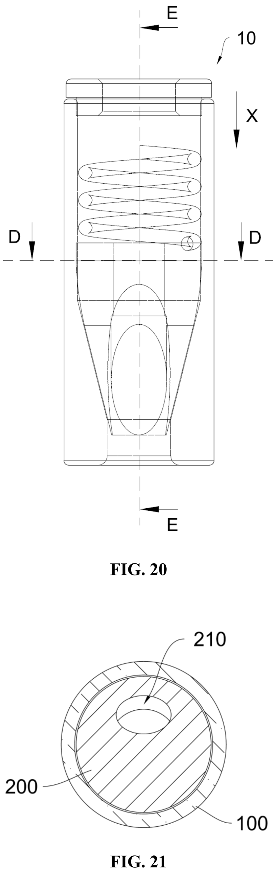

In some application scenarios, the locking device 10 is required to lock a single strand of medical wire 20. For example, in some suturing surgeries, the locking device 10 is adopted to lock one strand of suture. Accordingly, as shown in FIGS. 20 and 21, the locking member 200 can be provided with only one threading channel 210, and the medical wire 20 is correspondingly threaded through the threading channel 210. In this way, it is unnecessary to provide multiple threading channels 210 in the locking member 200, which can effectively reduce the volume of the locking member 200, and thus facilitate the miniaturization of the entire locking device 10.

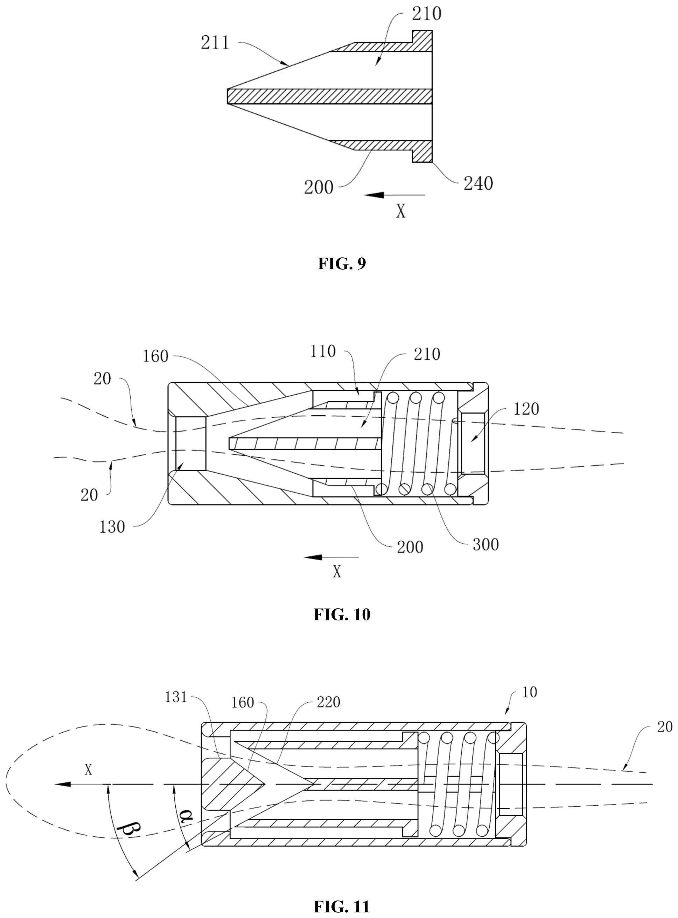

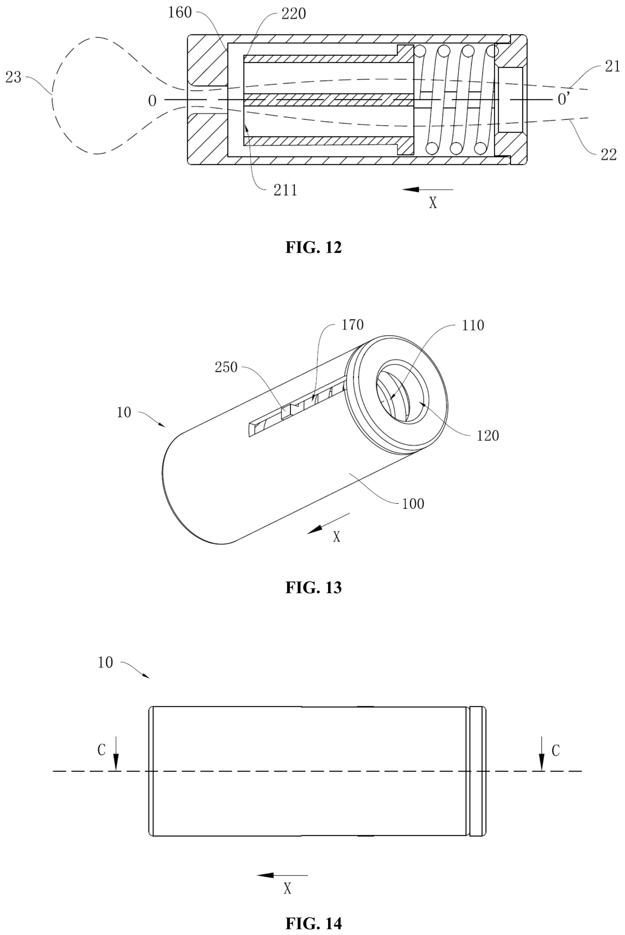

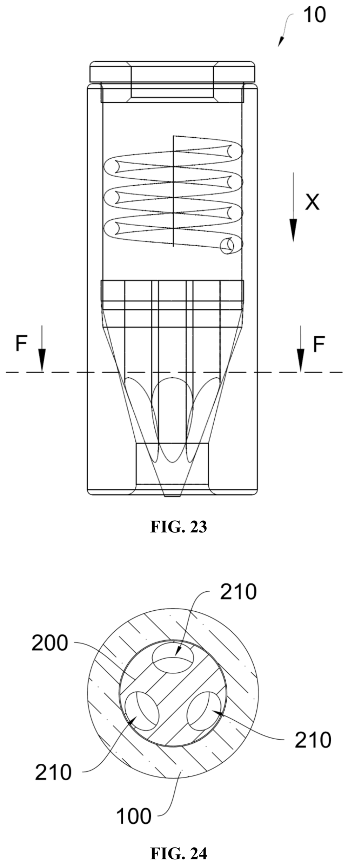

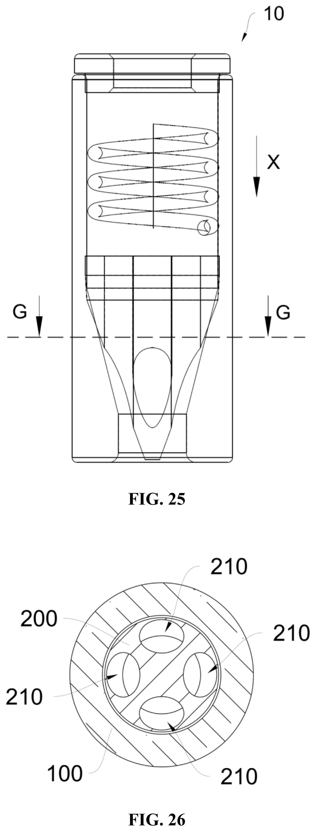

Referring to FIGS. 10, 11, and 12, in some application scenarios, the locking device 10 is required to lock multiple strands of medical wire 20, or to lock multiple pulling strand structures formed by a single strand of medical wire 20. For example, when ligating tumors or tissues under gastrointestinal conditions, it is necessary to tighten a wire loop 23 formed by the medical wire 20 (as shown in FIG. 12), and the first pulling strand 21 and the second pulling strand 22 of the medical wire 20 can be respectively threaded through the threading channel 210. Accordingly, the locking member 200 can be provided with multiple threading channels 210, with each threading channel 210 being capable of threading at least one strand of medical wire 20, so that the first pulling strand 21 can be threaded through one threading channel 210, and the second pulling strand 22 can be threaded through another threading channel 210. In the embodiment, the locking member 200 is provided to be subjected to an elastic force, and can press each medical wire 20 threaded in each threading channel 210 against the housing 100. Optionally, as shown in FIGS. 23, 24, 25, and 26, according to medical application requirements, three, four, or even more threading channels 210 can be provided.

Referring to FIGS. 10, 11, and 12, in some embodiments, the elastic member 300 is provided to apply an elastic force along a first direction X to the locking member 200, so that the locking member 200 can press the medical wire 20 against a first inner wall 160 of the housing 100. The first inner wall 160 is a portion of the cavity wall of the inner cavity 110. The threading channel 210 has a channel opening 211, and the channel opening 211 is formed on one side of the locking member 200 away from the elastic member 300. In the first direction X, at least a portion of the channel opening 211 is formed toward the first inner wall 160. That is to say, the locking member 200, being subjected to the elastic force of the elastic member 300 along the first direction X, can move along the first direction X or has a tendency to move along the first direction X. When the medical wire 20 is not threaded, the elastic member 300 can press the locking member 200 against the first inner wall 160, so that the portion of the channel opening 211 facing the first inner wall 160 is sealed by the first inner wall 160. After the medical wire 20 is threaded, the medical wire 20 located on the side of the channel opening 211 facing the first inner wall 160 can be pressed by the locking member 200 against the first inner wall 160, thereby being jointly pressed by the locking member 200 and the first inner wall 160 and firmly fixed.

Optionally, in the first direction X, the channel opening 211 can be completely formed toward the first inner wall 160, that is, the edge contour of the channel opening 211, when translated or projected along the first direction X, can fall on the first inner wall 160. The channel opening 211 can also be only partially formed toward the first inner wall 160, and the size of the portion of the channel opening 211 opposite to the first inner wall 160 can be specifically set according to the thickness of the medical wire 20. The thicker the medical wire 20 is, the smaller the opposite portion can be set; and the thinner the medical wire 20 is, the larger the opposite portion can be set. When the locking member 200 is subjected to the elastic force of the elastic member 300 and moves to the position closest to the first inner wall 160 (that is, in the inner cavity 110, the boundary position to which the locking member 200 can reach along the first direction X within its movable space), the distance between the edge contour of the channel opening 211 and the first inner wall 160 is smaller than the wire diameter of the medical wire 20, thereby achieving pressing fixation to the medical wire 20.

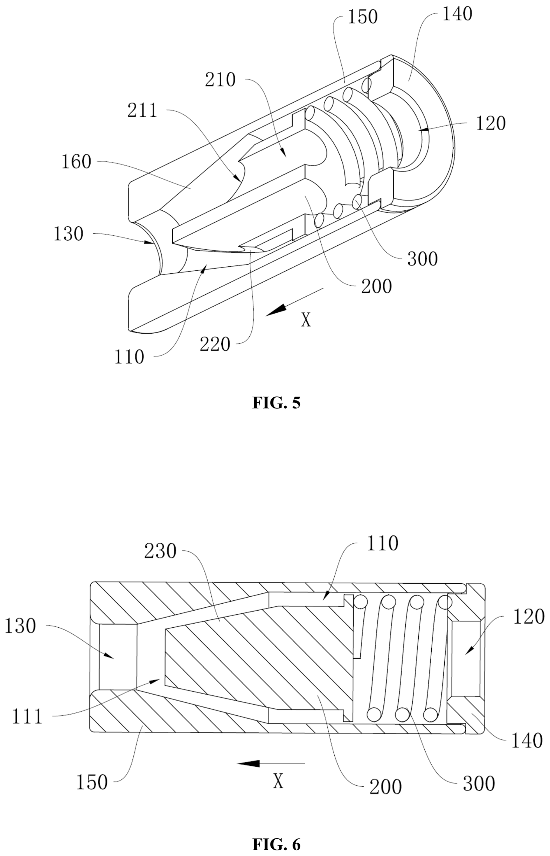

Referring to FIGS. 5, 8, 10, and 11, in some embodiments, the locking member 200 includes a first outer wall 220 away from the elastic member 300 and facing the first inner wall 160. The channel opening 211 is formed on the first outer wall 220, and both the first outer wall 220 and the first inner wall 160 are arranged to be inclined with respect to the first direction X. By arranging both the first outer wall 220 and the first inner wall 160, which are oppositely arranged, to be inclined with respect to the first direction X, the area of the opposite portions of the first outer wall 220 and the first inner wall 160 can be increased within a limited space, so that when the two press the medical wire 20, the contact area with the medical wire 20 is larger, the frictional force is greater, and the locking effect can be improved.

In some embodiments, the second threading hole 130 is located on one side of the locking member 200 away from the elastic member 300, and the first inner wall 160 is connected to an end of the hole wall of the second threading hole 130 close to the locking member 200. The first inner wall 160 can extend obliquely from the end of the hole wall of the second threading hole 130 close to the locking member 200 toward a direction away from the second threading hole 130 and close to the elastic member 300. When the medical wire 20 is pulled from the outside of the second threading hole 130, through the frictional force between the medical wire 20 and the first inner wall 160 and the first outer wall 220, the first outer wall 220 can be pulled toward the first inner wall 160, thereby further increasing the pressing force and the frictional force of the first inner wall 160 and the first outer wall 220 on the medical wire 20. Thus, the greater the force applied to the medical wire 20 outside the second threading hole 130, the greater the locking force of the locking device 10 on the medical wire 20, which is more favorable for improving the stability of locking. When the medical wire 20 is pulled in an opposite direction from the outside of the first threading hole 120, the acting force applied by the medical wire 20 to the locking member 200 is substantially opposite to the elastic force applied by the elastic member 300. When the pulling force is greater than the elastic force, the locking member 200 will be pushed by the medical wire 20 to move in a direction opposite to the first direction X, so that the locking member 200 moves away from the first inner wall 160, and the medical wire 20 can move freely.

Through the above arrangement, the locking device 10 can achieve one-way locking, so that the locking device 10 can be used for a ligation loop in a ligation surgery. As shown in FIG. 11, when the wire loop 23 is subjected to a force generated by tissue peristalsis or elasticity, the first pulling strand 21 and the second pulling strand 22 can be pulled, and the first pulling strand 21 and the second pulling strand 22 can further pull the locking member 200 closer, making the locking tighter and reducing the risk of loosening. A doctor can pull the first pulling strand 21 and/or the second pulling strand 22 to move the locking member 200 toward the elastic member 300, thereby pressing the elastic member 300 and reducing the pressing force on the first pulling strand 21 and the second pulling strand 22, and thus achieving the reduction of the wire loop 23.

In some embodiments, under the elastic force of the elastic member 300, one end of the locking member 200 away from the elastic member 300 can extend into the second threading hole 130 to occupy the internal space of the second threading hole 130, thereby pressing the medical wire 20 against the hole wall of the second threading hole 130 to improve the locking effect. In particular, when the locking device 10 is configured for a ligation loop in a ligation surgery, and when the wire loop 23 is subjected to a force generated by tissue peristalsis or elasticity, the first pulling strand 21 and the second pulling strand 22 can be pulled, and the first pulling strand 21 and the second pulling strand 22 can further pull the locking member 200 into the second threading hole 130, making the locking tighter. A doctor pulls the first pulling strand 21 and/or the second pulling strand 22 in an opposite direction, so that the locking member 200 moves toward the elastic member 300, so as to be separated from the second threading hole 130 and be further away from the first inner wall 160, thereby reducing the pressuring force applied to the first pulling strand 21 and the second pulling strand 22, and further achieving the reduction of the loop 23.

Referring to FIG. 11, in some embodiments, the housing 100 is provided with a plurality of second threading holes 130, and the number of the second threading holes 130 is consistent with the number of the threading channels 210. Each second threading hole 130 corresponds to one threading channel 210, and the corresponding second threading hole 130 and threading channel 210 are configured to thread the same medical wire 20, that is, the same medical wire 20 can sequentially pass through the corresponding second threading hole 130 and threading channel 210.

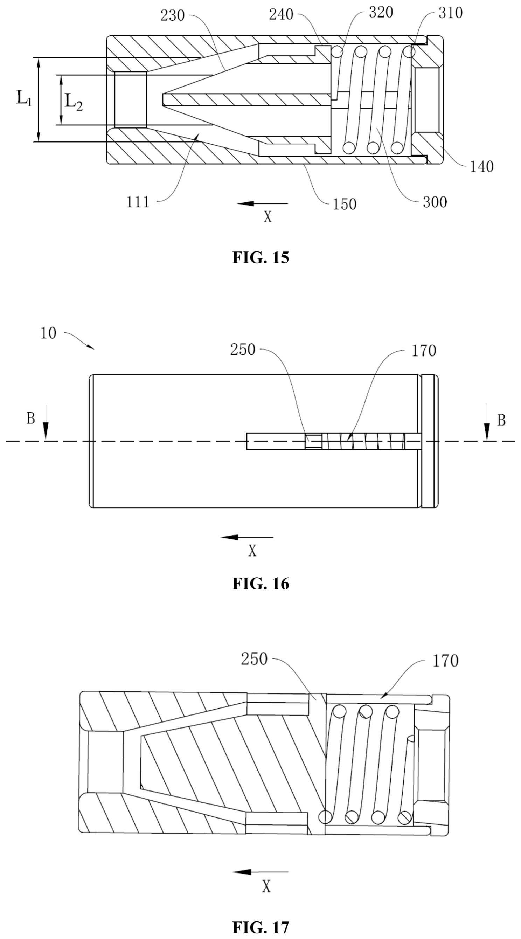

Referring to FIG. 10 and FIG. 15 together, in some embodiments, the inner cavity 110 is provided with a tapered section 111 formed by the first inner wall 160, and along the first direction X, the size Li of the tapered section 111 gradually decreases. The locking member 200 includes a tapered portion 230, at least portion of the tapered portion 230 is located inside the tapered section 111, and along the first direction X, the size L₂ of the tapered portion 230 gradually decreases, wherein the first outer wall 220 is arranged on the tapered portion 230. By providing the tapered section 111 in the inner cavity 110, and providing the tapered portion 230 on the locking member 200 corresponding to the tapered section 111, under the elastic force of the elastic member 300, the tapered portion 230 of the locking member 200 can be more compactly fitted with the first inner wall 160 surrounding the tapered section 111, thereby more stably locking the medical wire 20. When the medical wire 20 is subjected to a tensile force in a direction opposite to the elastic force, the locking member 200 can also more smoothly move backward to release the medical wire 20 and restore the unidirectional movement of the medical wire 20.

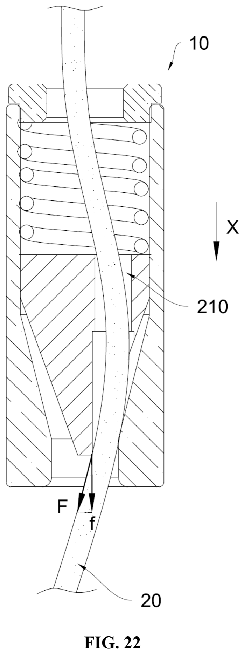

When the medical wire 20 is subjected to a tensile force toward the distal end, the frictional force F applied to the locking member 200 by the medical wire 20 is approximately as shown by the arrow in FIG. 22. The component f of the frictional force F in the first direction X can pull the locking member 200 toward the distal direction, thereby bringing the first outer wall 220 of the locking member 200 closer to the first inner wall 160 of the housing 100, so that the first outer wall 220 and the first inner wall 160 compress the medical wire 20 more tightly, which makes it more difficult to loosen, so that the medical wire 20 can be fixed more stably.

Referring to FIG. 11, in some embodiments, the angle between the first outer wall 220 and the first direction X is α, and the angle between the portion of the first inner wall 160 opposite to the first outer wall 220 and the first direction X is β, satisfying β≥α. That is to say, the inclination of the portion of the first inner wall 160 opposite to the first outer wall 220 is greater, so that the distance between the first outer wall 220 and the first inner wall 160 varies. The closer to the second threading hole 130 is, the smaller the distance is. Therefore, the medical wire 20 is clamped more tightly and the locking effect is improved.

Referring to FIG. 12, in some embodiments, the first inner wall 160 can be substantially perpendicular to the first direction X, the channel opening 211 is provided on the front end face of the locking member 200 away from the elastic member 300, and the front end face is the first outer wall 220. The elastic member 300 presses the locking member 200 so that the gap between the first outer wall 220 and the first inner wall 160 is smaller than the wire diameter of the medical wire 20, thereby the first outer wall 220 and the first inner wall 160 can clamp the medical wire 20 to lock and fix it.

In some embodiments, the threading channel 210 extends linearly along the first direction X, which on one hand facilitates processing and manufacturing, and on the other hand can reduce the volume of the locking member 200, thereby being advantageous for the miniaturization of the entire locking device 10. This is beneficial for medical applications, particularly for being easily delivered into the human body through the forceps channel of an endoscope, thus facilitating doctor operation.

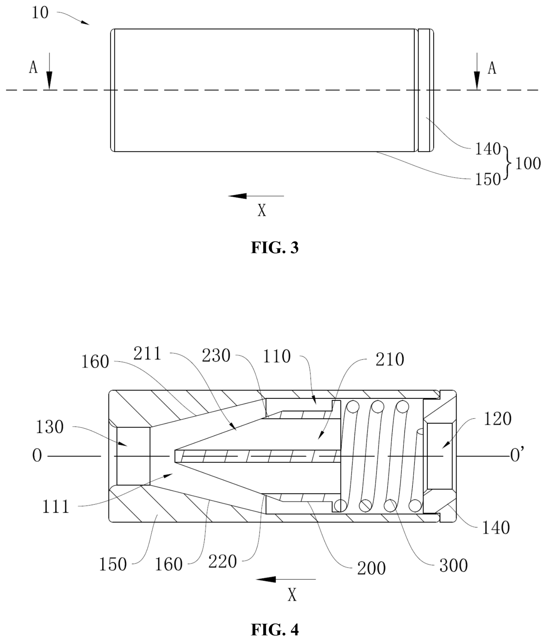

Referring to FIG. 4, in some embodiments, the first threading hole 120, the threading channels 210, and the second threading hole 130 are sequentially arranged along the first direction X, the second threading hole 130 has an axis OO' extending along the first direction X, and a plurality of threading channels 210 are arranged around the periphery of the axis OO'. In this embodiment, the locking device 10 can be provided with one second threading hole 130 and the plurality of threading channels 210, and the central axis of each threading channel 210 is not coaxial with the axis OO' of the second threading hole 130. Such an arrangement makes the threading paths of the medical wire 20 in the threading channel 210 and the second threading hole 130 offset and staggered relative to each other, so that the locking member 200 can more easily press the medical wire 20 against the first inner wall 160 on both sides of the second threading hole 130. In addition, by arranging the plurality of threading channels 210 around the periphery of the axis OO', it is possible to avoid interference among the threading channels 210, thereby improving the rationality of the spatial arrangement of the threading channels 210 on the locking member 200.

Referring again to FIG. 4, in some embodiments, the first threading hole 120, the inner cavity 110, and the second threading hole 130 are coaxially arranged along the first direction X, which facilitates threading the medical wire 20 and facilitates doctor operation.

Referring to FIGS. 13 to 17 together, in some embodiments, the housing 100 is provided with a first guiding portion 170, and the locking member 200 is provided with a second guiding portion 250 matched with the first guiding portion 170, the second guiding portion 250 and the first guiding portion 170 are in guiding cooperation to guide the locking member 200 along the first direction X. Through the guiding function of the first guiding portion 170 and the second guiding portion 250, when the locking member 200 is subjected to force and moves within the inner cavity 110, it can move stably along the first direction X, thereby improving the stability of the locking device 10 in locking the medical wire 20.

Optionally, among the first guiding portion 170 and the second guiding portion 250, one is provided as a guiding groove, and the other is provided as a guiding protrusion, and the guiding protrusion is movably provided within the guiding groove. For example, in the embodiment shown in FIG. 13, the first guiding portion 170 is a guiding groove formed on the housing 100, the guiding groove extends along the first direction X and communicates with the inner cavity 110. The guiding groove can, as shown in FIG. 13, penetrate the housing 100 in the thickness direction of the housing 100, or can be formed on the side of the housing 100 facing the inner cavity 110 without penetrating the housing 100. The second guiding portion 250 is a guiding protrusion protrudingly provided on the locking member 200, and the guiding protrusion is arranged within the guiding groove and capable of moving along the guiding groove. Thus, as shown in FIG. 17, when the locking member 200 moves under the elastic force applied by the elastic member 300 or under the tensile force applied by the medical wire 20, it can stably move along the first direction X. For another example, the first guiding portion 170 can be a guiding protrusion, and the guiding protrusion can protrude from the inner wall of the housing 100 toward the inner cavity 110. The second guiding portion 250 is a guiding groove, and the guiding groove is formed on the locking member 200 and extends along the first direction X. Through cooperation with the guiding protrusion, the locking member 200 can also move stably along the first direction X.

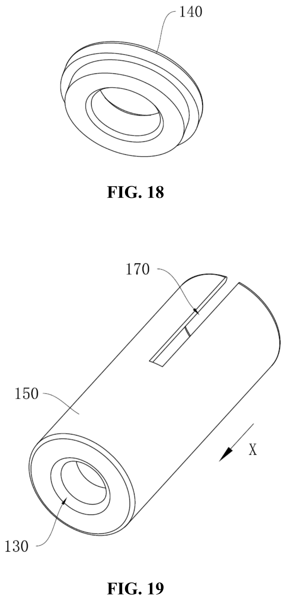

Referring to FIGS. 2, 3, 4, 18, and 19, in some embodiments, the housing 100 includes a cover portion 140 and a main body portion 150, the cover portion 140 and the main body portion 150 are connected to form the inner cavity 110, the first threading hole 120 is provided on the cover portion 140, and the second threading hole 130 is provided on the main body portion 150. By providing the housing 100 into a split structure including the cover portion 140 and the main body portion 150, the assembly of the locking device 10 can be facilitated, and the replacement and maintenance of components can be more convenient.

Optionally, the elastic member 300 has a first end 310 and a second end 320 opposite to each other along the first direction X, the first end 310 abuts against the cover portion 140, and the second end 320 abuts against the locking member 200. Thus, the elastic member 300 can apply an elastic force to the locking member 200 along the first direction X. The elastic member 300 can be a spring or another structure having elasticity.

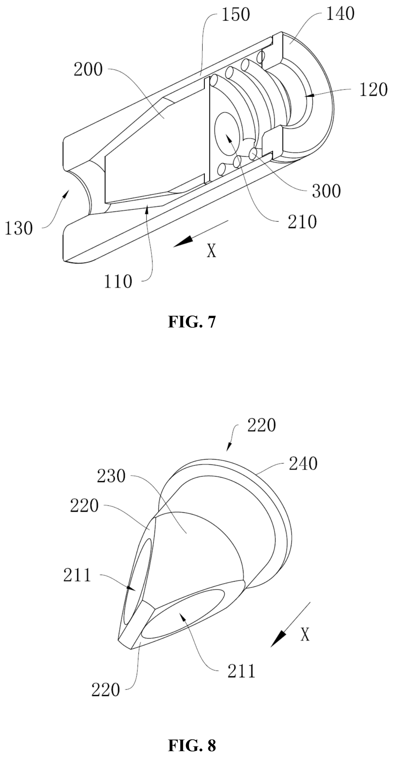

Referring to FIG. 8, in some embodiments, the locking member 200 is provided with a flange portion 240, and the second end 320 abuts against the side wall of the flange portion 240 facing the cover portion 140. By providing the flange portion 240, the connection and assembly between the elastic member 300 and the locking member 200 can be facilitated, and the flange portion 240 can contact and guide along the inner wall of the housing 100, thereby improving the stability of the locking member 200 when moving inside the inner cavity 110. Optionally, the second guiding portion 250 described above can be protrudingly provided on the flange portion 240.

Optionally, the housing 100 can be in a cylindrical shape, a prismatic shape, or an irregular shape, which can be selected according to actual medical application requirements.

Accordingly, the present invention further provides a tissue ligation apparatus, and the tissue ligation apparatus can be applied to operations such as tumor, tissue ligation, and vessel closure in the gastrointestinal environment. Referring again to FIGS. 10, 11, and 12, the tissue ligation apparatus includes the locking device 10 according to any of the above embodiments and a medical wire 20, wherein the medical wire 20 can be one or more strands of nylon wires, metal wires, or other medical material wires. The medical wire 20 is threaded through the first threading hole 120, the threading channel 210, and the second threading hole 130 of the locking device 10.

It can be understood that the tissue ligation apparatus can include all the technical features and technical effects of the locking device 10 described above, and therefore, details will not be repeated herein.

Referring again to FIGS. 11 and 12, the medical wire 20 includes a first pulling strand 21, a second pulling strand 22, and a wire loop 23. The first pulling strand 21 is threaded through the first threading hole 120, one threading channel 210, and the second threading hole 130. The second pulling strand 22 is threaded through the first threading hole 120, another threading channel 210, and the second threading hole 130. The wire loop 23 is arranged on a side of the second threading hole 130 away from the threading channel 210 and connects to the first pulling strand 21 and the second pulling strand 22. When the medical wire 20 is pulled in the opposite direction from the outside of the first threading hole 120, the acting force applied by the medical wire 20 to the locking member 200 is substantially opposite to the elastic force applied by the elastic member 300. When the pulling force is greater than the elastic force, the locking member 200 will be pushed by the medical wire 20 to move in a direction opposite to the first direction X, so that the locking member 200 moves away from the first inner wall 160, and the medical wire 20 can move in one direction. The doctor can pull the first pulling strand 21 and/or the second pulling strand 22 to reduce the size of the wire loop 23, thereby tightening the tissue or tumor.

It should also be noted that the tissue ligation device can further include structures such as an operation handle for operating the first pulling strand 21 and/or the second pulling strand 22 described above.

Accordingly, an endoscopic surgical instrument is provided. The endoscopic surgical instrument includes the locking device 10 according to any of the embodiments described above, or includes the tissue ligation apparatus according to any of the embodiments described above. Therefore, the endoscopic surgical instrument can include all the technical features and technical effects of the locking device 10 or the tissue ligation apparatus described above, and details will not be repeated herein.

In the above embodiments, the descriptions of various embodiments focus on different aspects, and parts not described in detail in one embodiment may be referred to in the relevant descriptions of other embodiments.

The above provides a detailed introduction of the locking device, tissue ligation apparatus, and endoscopic surgical instrument provided in the embodiment of the present invention, and specific examples have been used to illustrate the principles and embodiments of the present invention. The description of the above embodiments is only intended to help in understanding the technical solutions and the core ideas of the present invention. Those of ordinary skill in the art should understand that one may still modify the technical solution described in the foregoing embodiments, or make equivalent substitutions for some technical features therein. These modifications or substitutions do not depart from the essence of the corresponding technical solution within the scope of the technical solution of the embodiments of the present invention.

## Claims

1. A locking device, configured to lock a medical wire (20), wherein the locking device (10) comprises:
a housing (100) provided with an inner cavity (110), a first threading hole (120), and
a second threading hole (130), wherein the first threading hole (120) and the second threading hole (130) are respectively in communication with the inner cavity (110);
a locking member (200) arranged in the inner cavity (110), wherein the locking member (200) is provided with a threading channel (210), and the first threading hole (120), the threading channel (210), and the second threading hole (130) are configured for the medical wire (20) to pass through; and
an elastic member (300) arranged in the inner cavity (110) and connected with the housing (100) and the locking member (200) respectively, wherein the elastic member (300) is arranged to apply an elastic force to the locking member (200), so that the locking member (200) is able to press the medical wire (20) against the housing (100).

2. The locking device according to claim 1, wherein the locking member (200) is provided with a plurality of threading channels (210), and the locking member (200) is provided to be subjected to the elastic force so as to press the medical wire (20) threaded in each of threading channels (210) against the housing (100).

3. The locking device according to claim 1 or 2, wherein the elastic member (300) is arranged to apply an elastic force along a first direction (X) to the locking member (200), so that the locking member (200) is able to press the medical wire (20) against a first inner wall (160) of the housing (100); and
each of the threading channels (210) is provided with a channel opening (211), the channel opening (211) is formed on one side of the locking member (200) away from the elastic member (300), and in the first direction (X), at least a portion of the channel opening (211) is formed toward the first inner wall (160).

4. The locking device according to claim 3, wherein the locking member (200) comprises a first outer wall (220) away from the elastic member (300) and facing the first inner wall (160), the channel opening (211) is arranged on the first outer wall (220), and both the first outer wall (220) and the first inner wall (160) are inclined relative to the first direction (X).

5. The locking device according to claim 4, wherein the second threading hole (130) is located on one side of the locking member (200) away from the elastic member (300); and
the first inner wall (160) is arranged on one side of a hole wall (131) of the second threading hole (130) close to the locking member (200) and is connected with the hole wall (131), and the first inner wall (160) extends obliquely in a direction away from the second threading hole (130) and close to the elastic member (300).

6. The locking device according to claim 5, wherein the locking member (200) is provided to be subjected to the elastic force, and one end of the locking member (200) away from the elastic member (300) is capable of at least partially extending into the second threading hole (130).

7. The locking device according to claim 2, wherein the housing (100) is provided with a plurality of second threading holes (130), and the number of the second threading holes (130) corresponds to the number of the threading channels (210).

8. The locking device according to claim 4, wherein the inner cavity (110) has a tapered section (111) formed by the first inner wall (160), and along the first direction (X), a size L1 of the tapered section (111) gradually decreases;
the locking member (200) comprises a tapered portion (230), at least a portion of the tapered portion (230) is located within the tapered section (111), and along the first direction (X), a size L2 of the tapered portion (230) gradually decreases, with the first outer wall (220) arranged on the tapered portion (230), wherein
an angle between the first outer wall (220) and the first direction (X) is α, and an angle between a portion of the first inner wall (160) opposite to the first outer wall (220) and the first direction (X) is β, satisfying β≥α.

9. The locking device according to claim 3, wherein the threading channels (210) extend linearly along the first direction (X), and/or
the first threading hole (120), the threading channels (210), and the second threading hole (130) are sequentially arranged along the first direction (X), the second threading hole (130) has an axis (OO') extending along the first direction (X), and the plurality of threading channels (210) are arranged around a periphery of the axis (OO').

10. The locking device according to claim 9, wherein the first threading hole (120), the inner cavity (110), and the second threading hole (130) are coaxially arranged along the first direction (X).

11. The locking device according to claim 1, wherein the housing (100) is provided with a first guiding portion (170), and the locking member (200) is provided with a second guiding portion (250) matched with the first guiding portion (170), the second guiding portion (250) and the first guiding portion (170) are in guiding cooperation to guide the locking member (200) along the first direction (X), wherein
among the first guiding portion (170) and the second guiding portion (250), one is provided as a guiding groove, the other is provided as a guiding protrusion, and the guiding protrusion is movably provided within the guiding groove.

12. The locking device according to claim 1, wherein the housing (100) comprises a cover portion (140) and a main body portion (150), the cover portion (140) and the main body portion (150) are connected to form the inner cavity (110), the first threading hole (120) is provided in the cover portion (140), and the second threading hole (130) is provided in the main body portion (150).

13. The locking device according to claim 12, wherein the elastic member (300) has a first end (310) and a second end (320) opposite to each other along the first direction (X), the first end (310) abuts against the cover portion (140), and the second end (320) abuts against the locking member (200).

14. The locking device according to claim 13, wherein the locking member (200) is provided with a flange portion (240), and the second end (320) abuts against a side wall of the flange portion (240) facing the cover portion (140).

15. A tissue ligation apparatus, comprising:
the locking device (10) according to any one of claims 1 to 18; and
a medical wire (20), wherein the medical wire (20) is threaded through the first threading hole (120), the threading channel (210), and the second threading hole (130)
of the locking device (10), wherein
the medical wire (20) comprises:
a first pulling strand (21) threaded through the first threading hole (120), one of the threading channels (210), and the second threading hole (130);
a second pulling strand (22) threaded through the first threading hole (120), another of the threading channels (210), and the second threading hole (130); and
a wire loop (23) arranged on one side of the second threading hole (130) away from the threading channels (210) and connecting to the first pulling strand (21) and the second pulling strand (22).
